# EUROPÄISCHE PATENTSCHRIFT

(11) **EP 0 307 604 B1**
(45) Veröffentlichungstag und Bekanntmachung des Hinweises auf die Patenterteilung: **27.04.1994**
(21) Anmeldenummer: 88112691.6
(22) Anmeldetag: 04.08.1988
(51) Int. Cl.: A61B 3/02

(54) **Vorrichtung und Verfahren zur Auffindung von Skotomen im Auge eines Probanden**
Means and method for tracing scotomas in the eye of a probationer
Dispositif et méthode pour trouver les scotomes dans l'oeil d'un patient

(30) Priorität: 18.09.1987 DE 3731415
(43) Veröffentlichungstag der Anmeldung: 22.03.1989
(73) Patentinhaber: Oculus Optikgeräte GmbH, 35582 Wetzlar (DE)
(72) Erfinder: Aulhorn, Elfriede, Prof., D-7400 Tübingen (DE); Köst, Gert, D-7400 Tübingen (DE)
(74) Vertreter: Missling, Arne, Dipl.-Ing.

(56) Entgegenhaltungen:
- WO-A-85/02103
- US-A- 4 334 738
- US-A- 4 634 243

## Beschreibung

Die Erfindung betrifft ein Verfahren zur Auffindung von Skotomen im Auge eines Probanden.

Bei der Gesichtsfelduntersuchung wird die Lichtunterschiedsempfindlichkeit pro Netzhautort geprüft. Man kennt dabei zwei verschiedene Methoden und zwar einmal die kinetische Perimetrie, bei der mit bewegter Prüfmarke untersucht wird, während bei der statischen Perimetrie, an einem fixen Ort die Lichtunterschiedsempfindlichkeit von dunklen Leuchtdichten her kommend exakt ausgemessen wird. Heute werden vielfach computergestützte Systeme eingesetzt, die nach der sogenannten Rasterperimetrie-Methode arbeiten. Dabei wird nach einem Programm ein Prüfpunktraster automatisch angefahren, entweder mit angepaßter Prüfpunkthelligkeit oder mit exakter Schwellenmessung, wobei dann die Ergebnisse gespeichert und am Ende einer Untersuchung ausgedruckt werden. Orte, bei denen keine Wahrnehmung oder eine herabgesetzte. Lichtunterschiedsempfindlichkeit vorliegt, werden gesondert gekennzeichnet. Für die Behandlungserfolgsaussicht ist es desto günstiger, je früher die Ausfälle festgestellt werden.

Für die Gesichtsfelduntersuchungen werden zumeist Kugelperimeter verwendet, bei denen entweder der Prüfpunkt in die Kugel über ein verstellbares Projektionssystem projiziert wird oder aber Dioden- oder Glasfaserlichtleiter in der Kugel montiert sind. Diese bekannten Systeme sind alle recht aufwendig und mechanisch sehr empfindlich, da höchste Ansprüche an die Genauigkeit gestellt werden.

Aus der US-PS 4 634 243 ist ein Verfahren zur Gesichtsfeldutersuchung bekannt, bei welchem dem Probanden ein Rauschmuster-Bild gezeigt wird, in welchem eine regelmäßige Anordnung von Bildpunkten in Form eines geometrisch gleichförmigen, begrenzten Musters enthalten ist. Dieses im Vergleich zur Gesamtfläche des Bildes kleine Muster weist regelmäßig angeordnete Punkte gleicher Dichte auf, welche sich sowohl von der Anordnung als auch von der Dichte der Punkte des Rauschbildes unterscheiden. Zur Ermittlung von Skotomen oder ähnlichen Sehstörungen wird dieses geometrisch angeordnete Punktmuster über das gesamte Bild verschoben. Bei diesem Verfahren wird auf dem Schirm der Stimulus aus Bildpunkten mit konstanter Helligkeit in statistisch verteilten Bildschirmpunkten eingeblendet, wobei ein hundertprozentiger Gesichtsfeldausfall dann gegeben ist, wenn der eingeblendete Stimulus mit konstanter Helligkeit nicht erkannt wird. Es erfolgt somit eine Abtastung des zentralen Gesichtsfeldes des Probanden bis 30°. Auch dieses Verfahren erweist sich als nachteilig, da mittels aufwendiger und langdauernder Untersuchungen eine Abtastung des gesamten Gesichtsfeldes erforderlich ist.

Ein Problem bei allen diesen Gesichtsfelduntersuchungen besteht darin, daß der Proband selber nicht feststellen kann, ob Gesichtsfeldausfälle vorliegen oder nicht. Bei jeder Untersuchung muß also die gesamte Netzhaut untersucht werden. Die Untersuchung wäre wesentlich einfacher und in kürzerer Zeit durchzuführen, wenn bei Beginn der Untersuchung bereits bekannt wäre, wo derartige Gesichtsfeldausfälle zu erwarten sind.

Der Erfindung liegt die Aufgabe zugrunde, eine Vorrichtung und ein Verfahren zur Auffindung von Skotomen im Auge eines Probanden vorzuschlagen, das es dem Probanden gestattet, in kürzester Zeit derartige Gesichtsfeldausfälle selbst anzugeben.

Hinsichtlich der Vorrichtung wird die Aufgabe durch die Merkmalskombination des Anspruches 1 hinsichtlich des Verfahrens durch die Merkmale des Anspruches 11 gelöst.

Gemäß der Erfindung wird somit dem Auge des Probanden auf einem Schirm ein Bild angeboten, das aus einer Vielzahl dicht nebeneinanderliegender Punkte besteht, deren Helligkeit laufend wechselt, wobei das Bild zusätzlich einen Fixationspunkt für das Auge aufweist. Der Wechsel der Helligkeit der einzelnen Bildpunkte kann regellos oder auch periodisch erfolgen. Als einfachstes Gerät für die Durchführung der perimetrischen Untersuchungen hat sich ein hochauflösender Videomonitor erwiesen, der eine regellos flimmernde Fläche mit einem sehr kleinen Korn aufweist, ähnlich einem Rauschfeld, wie es sich ergibt, wenn eine Störung bei Fernsehern eintritt. Das Auge des Probanden befindet sich in einem vorgegebenen Abstand zum Videoschirm, wobei das Auge auf einen bestimmten Fixationspunkt am Bildschirm fixiert ist. Der Proband kann bei Betrachtung des Rauschbildes in Sekundenschnelle feststellen, an welchen Stellen Skotome vorliegen.

Die beschriebenen krankhaften Skotome können deutlich sichtbar gemacht werden, wenn der Patient statt auf eine homogene Fläche auf eine mit kleinem Korn in hoher Frequenz flimmernde Hell-Dunkel-Fläche blickt, ähnlich der des Rauschfeldes am eingeschalteten Fernsehmonitor, wenn kein Programm empfangen wird. Wenn der Blick des Patienten durch einen in die Mitte des Videoschirmes aufgeklebten, oder programmiert eingeblendeten, deutlich sichtbaren Punkt fixiert ist, wird das Skotom als weniger oder gar nicht flimmernde umschriebene Fläche empfunden, deren Helligkeit sich deutlich von der Umgebung abhebt. Der Beobachter kann die Skotomgrenzen mit dem Finger genau auf dem Videoschirm nachzeichnen. Dieses Markieren der eigenen Skotome geschieht meist spontan ohne Aufforderung, weil der Eindruck der begrenzten "Wolke" im Rauschfeld für den Patienten so eindrucksvoll ist. Alle umschriebenen absoluten Skotome, die durch Schädigung der Netzhaut, des Sehnerven, des Chiasmas oder des Traktus opticus entstanden sind, können in gleicher Weise von den Patienten angegeben werden, wie glaukombedingte Ausfälle - wobei vorläufig allerdings noch nicht erkennbar ist, wann ein Skotom als hellere und wann als dunklere Wolke wahrgenommen wird. Aber in jedem Fall wird das Skotom dadurch bemerkt, daß in seinem Bereich das Flimmern des Rauschfeldes nicht oder doch geringer als in der Umgebung empfunden wird.

Voraussetzung für eine reproduzierbare Wahrnehmung der Skotome durch den Patienten und eine schnelle perimetrische Kontrolle und Registrierung des wahrgenommenen Gesichtsfeldausfalles ist ein Video-Monitor, auf dem sowohl ein standartisiertes, von einem Computer erzeugtes, nur in seinen Parametern veränderliches Rauschfeld, wie auch eine konturlose Fläche mit einem frei beweglichen, nach Wahl hellen oder dunklen Prüfpunkten erscheinen kann. Des weiteren ist es vorteilhaft, zur Erkennung der Lage und Ausdehnung der Ausfälle auf dem Video-Monitor ein Gesichtsfeldschema mit Gradangaben einzublenden.

Die Skotomwahrnehmung im Rauschfeld kann auf sehr einfache Weise mit einer messenden perimetrischen Untersuchung auf dem Schirm desselben Monitors als Testfeld verbunden werden. Dabei spielt die Skotomwahrnehmung im Rauschfeld die Rolle eines ersten orientierenden Tests, also eines Screeningverfahrens, das den Augenarzt in Sekundenschnelle darüber informiert, ob und wo Ausfälle im 30 Grad Gesichtsfeld vorhanden sind. Anschließend kann dann im Skotombereich eine manuelle kinetische Perimetrie herkömmlicher Art oder eine automatische Rasterperimetrie angeschlossen werden. - Der Vorteil einer solchen zweiseitigen Methode liegt darin, daß die messende Perimetrie nur in Ausfallsbereichen durchgeführt zu werden braucht, was gegenüber den bisherigen Methoden eine erhebliche Zeiteinsparung bedeutet.

Die gleichzeitige Verwendung eines hochauflösenden Video-Monitors sowohl zur Rauschfeld- als auch zur konventionellen Perimetrie erlaubt eine exakte Vergleichbarkeit der gewonnenen Untersuchungsergebnisse. Bei beiden Untersuchungsarten können in gleicher Weise Kontrast, Helligkeit und Farbe geändert werden. Hierbei kann die kinetische wie auch statische Perimetrie entweder freibeweglich von Hand oder aber auch automatisch durchgeführt werden. Die Art campimetrischen Vorgehens kann dabei ganz der Form und Größe der zuvor mit der Rauschfeldperimetrie aufgedeckten Gesichtsfeldausfälle angepaßt werden. Auf diese Weise können die Vorteile der sekundenschnellen Entdeckung von Skotomen mit der Exaktheit einer der Situation angepaßten campimetrischen Methode verbunden werden.

In Abhängigkeit der Größe der Video-Monitore ergibt sich der untersuchte Gesichtsfeldbereich. Dieser Gesichtsfeldbereich leigt bei den normalen Video-Monitoren bei 60 Grad. Dieser Gesichtsfeldbereich kann jedoch durch Verschieben des Fixationspunktes vergrößert werden.

Als besonders vorteilhaft hat sich herausgestellt, wenn des Rauschbild in einer Perimeterhalbkugel projiziert wird, denn dann können die Vorteile der zweigleisigen perimetrischen Methode auch in Form einer Halbkugelperimetrie durchgeführt werden.

Bei der erfindungsgemäßen Vorrichtung erweist es sich als besonders vorteilhaft, wenn ein Helligkeitsabgleich des Bildes bzw. des Bildschirms in einem bestimmten Meßpunkt vorgenommen werden kann. Dieser Abgleich kann beispielsweise mit Hilfe einer Photodiode erfolgen, wobei der durch die Photodiode fließende Strom als Maß für die Helligkeit des Bildschirms dient. Der Abgleich kann sowohl laufend während der Untersuchung, als auch in regelmäßigen Abständen vorgenommen werden. Es ist somit möglich, präzise Leuchtdichteabstufungen einstellen und überprüfen zu können, um reproduzierbare Untersuchungsergebnisse zu erzielen. Erfindungsgemäß ist es auch möglich, den Helligkeitsabgleich an mehreren Bildpunkten vorzunehmen.

Mittels der erfindungsgemäßen Vorrichtung ist es auch möglich, nur die Bereiche des Gesichtsfeldes mit einem Rauschmuster zu belegen, welche vom Probanden bereits als Bereiche mit Sehstörungen erkannt wurden. Wenn das übrige Bild kein Rauschen zeigt und der Proband erklärt, das gesamte Bild sei ohne Rauschmuster, dann ist der Gesichtsfeldausfall positiv nachgewiesen. Um das Rauschbild nur auf der vorbestimmbaren Fläche erscheinen zu lassen, ist die Vorrichtung mit einem Lichtgriffel, einer Maus oder einem Touchscreen versehen.

Erfindungsgemäß ist vorgesehen, zur Einjustierung des Fixationspunktes das Bild zu verschieben, um den Fixationspunkt an die Stellung des Auges des Probanden anzupassen. Diese Verschiebung kann mittels eines Rechenprogramms erfolgen, wobei dem Probandenauge beispielsweise ein Koordinatensystem angeboten wird. Diese Justiermöglichkeit bietet den Vorteil, daß auf die bisher bekannten, aufwendigen Vorrichtungen zur Fixation und Einstellung des Kopfes des Probanden verzichtet werden kann. Weiterhin ist die Einstellung des Fixationspunktes wesentlich schneller und einfacher durchführbar.

Erfindungsgemäß ist es möglich, die Bildpunkte in ihrem Durchmesser zu verändern, um fehlsichtigen Probanden das Erkennen der einzelnen Bildpunkte zu ermöglichen. Weiterhin ist es erfindungsgemäß möglich, verschiedene Farben der Bildpunkte oder Farbänderungen vorzusehen, um zu verhindern, daß der Proband das Auge an das Rauschbild adaptiert. Weiterhin ist es möglich, die Frequenz des Helligkeitswechsels der Bildpunkte zu verändern, um eine Gewöhnung des Patienten auszuschließen. Bei einer farblichen Veränderung der Bildpunkte ist es zugleich möglich, das Farbsehen des Probanden zu testen.

Mittels der erfindungsgemäßen Vorrichtung ist es durch eine positiv-negativ Umstellung der Helligkeit der einzelnen Bildpunkte möglich, die Lage der Skotome nachzuprüfen.

Zur Erleichterung der Fixation des Auges des Patienten ist es mittels der erfindungsgemäßen Vorrichtung möglich, in dem Computergesteuerten Bild beispielsweise Zahlenreihen einzublenden, wobei die Festlegung des Fixationspunktes dadurch erfolgen kann, daß der Proband angibt, welche Zahl oder welche Zahlen diese Zahlenreihen er sieht. Eine Kontrolle der Fixation kann dadurch erfolgen, daß der blinde Fleck des Probanden, auf welchem er nichts sieht mit einem Fixationspunkt oder Fixationsbereich in Übereinstimmung gebracht wird. Hierbei sollte die Größe dieses Fixationspunktes oder Fixationsbereiches, d.h. eines durch den Computer erzeugten Ausfallbildes geringfügig kleiner sein, als der blinde Fleck selbst. Der Proband wird dadurch bereits bei einer geringfügigen Änderung der Fixation des Auges eine Abweichung sofort feststellen können.

Ein weiterer Vorteil der erfindungsgemäßen Vorrichtung liegt darin, daß auf dem Bild eine Vielzahl von Grauwerten in feinsten Abstufungen darstellbar sind, beispielsweise bei Verwendung eines üblichen Fernsehschirmes 256 Grauwerte.

Es ist somit auf besonders einfache Weise möglich, Schwellenmessungen durchzuführen.

Eine besonders günstige Weiterbidlung der erfindungsgemäßen Vorrichtung ist dadurch gegeben, daß der behandelnden Person beispielsweise dem Arzt ein zweiter Schirm zugeordnet ist, so daß dieser jederzeit über die Lage und Größe der jeweiligen Ausfallgebiete informiert ist.

Hinsichtlich des erfindungsgemäßen Verfahrens erweist es sich als besonders vorteilhaft, zur frühen Erkennung von Glaukomen eine künstliche Erhöhung des Augendruckes beim Probanden zu bewirken. Dies kann beispielsweise über eine Saugpumpe oder medikamentös erfolgen. Auf diese Weise ist es möglich, die Sehfehler vorherzubestimmen, welche bei einer natürlichen Veränderung des Augendruckes auftreten würden.

Die leichte Wahrnehmbarkeit der eigenen Gesichtsfeldausfälle im Rauschfeld hat noch weitere Vorteile. Sie ermöglicht es, daß interessierte Patienten ihre Skotome zu Hause am Fernsehschirm selbst kontrollieren können. Sie müssen nur darüber unterichtet werden, daß sie stets den gleichen Abstand zum Schirm einhalten müssen (ca. 30 cm), ein Auge abdecken müssen und daß außerdem eine ruhige Fixation mit Hilfe eines Fixierpunktes gewährleistet sein muß. Eine eventuelle Vergrößerung der absoluten Skotome oder das Auftreten neuer Skotome kann dann von gut beobachtenden Patienten zu Hause am eigenen Fernsehgerät festgestellt werden.

Im Folgenden wird die Erfindung anhand eines Ausführungsbeispiels in Verbindung mit der Zeichnung geschrieben, wobei sich weitere Vorteile der Erfindung aus dieser Beschreibung ergeben. Dabei zeigt:
- Fig. 1: eine schematische Seitenansicht eines Ausfuhrungsbeispiels der erfindungsgemäßen Vorrichtung,
- Fig. 2: eine Schnittansicht entlang dr Linie II-II von Fig. 1,
- Fig. 3: eine Darstellung der praktischen Vorgehensweise bei der Untersuchung und
- Fig. 4: in schematischer Darstellung eine Vorrichtung zur Erzeugung eines Videobildes für die Schwellenperimetrie.

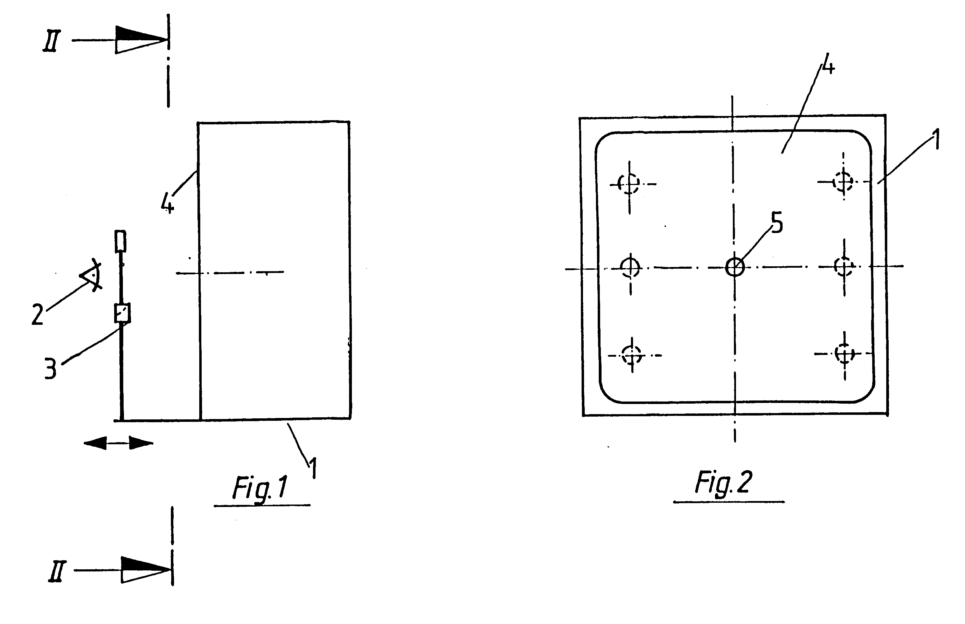

In Fig. 1 ist der Video-Monitor 1 in Seitenansicht dargestellt. Zur Herstellung eines exakten und definierten, jedoch veränderbaren Abstandes des Probanden mit seinem Auge 2 vom Video-Monitor 1 dient eine Kinnstütze 3, die in einer Entfernung vom Schirm 4 des Video-Monitors 1 angeordnet ist, so daß in etwa der zu untersuchende Gesichtsfeldberich einen Durchmesser von 60 Grad aufweist.

Fig. 2, die eine Ansicht des Schirmes 4 wiedergibt, zeigt einen Fixationspunkt 5, der normalerweise in der Mitte des Schirmes angeordnet ist. Dieser Fixationspunkt kann jedoch seitlich und in der Höhe verschoben werden, so daß hierdurch der Gesichtsfeldbereich zum Rand hin verschoben und somit vergrößert werden kann. Eine Verschiebung des Fixationspunktes ist dann immer entbehrlich, wenn der Video-Monitor eine derartige Größe aufweist, daß das gesamte Gesichtsfeld des Probanden erfaßt werden kann.

Bei der praktischen Anwendung hat sich herausgestellt, daß die Skotome am deutlichsten wahrnehmbar sind, wenn das Rauschfeld mit hoher Flimmerfrequenz, hohen Kontrasten und kleinem Korn dargeboten wurde. Aus diesem Grunde wurden die meisten Untersuchungen bei einer Frequenz von 50 Hz, einer Leuchtdichte von 60 cd/m² der hellen Elemente und 0,8 cd/m² der dunklen Elemente durchgeführt. Das Verhältnis der dunklen zu den hellen Elementen betrug 50% oder 70% zugunsten der dunklen Elemente. Die Elemente weisen eine Größe von 15 Winkelminuten in Form von kleinen Quadraten auf.

Bei der in Fig. 1 dargestellten Anordnung weist der Kopf des Patienten einen Abstand von ca. 30 cm von dem Monitor auf. Das Testfeld des Monitors ist beispielsweise 25 auf 38 cm groß, so daß sich ein Gesichtsfeldausschnitt von ca. 35 Grad in der Horizontalen und ca. 24 Grad in der Vertikalen ergibt. Es ist mittels der erfindungsgemäßen Vorrichtung möglich, ein Polarkoordinatensystem mit Kreisen in 5 Grad Sehwinkelabstand auf dem Monitor einzublenden. Erfindungsgemäß können auf dem Testfeld nicht zur das Rauschbild und das Polarkoordinatenschema erscheinen, es ist vielmehr auch möglich, ein homogenes Feld für die herkömmliche Perimetrie darzustellen. Dieses Feld kann in verschiedenen Leuchtdichten zwischen 60 und 0,8 cd/m² angeboten werden.

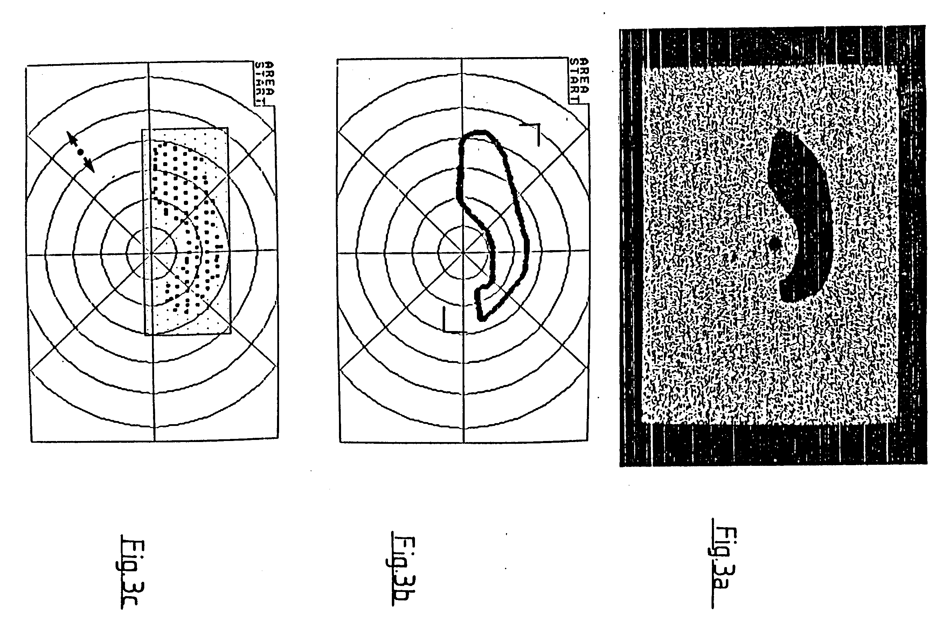

Im Folgenden wird an Hand der Figuren 3a, 3b und 3c ein erfindungsgemäßes Untersuchungsverfahren beschrieben.Die Untersuchung beginnt mit der Darbietung des Rauschfeldes auf dem Monitor (Fig. 3a). Nach Fixierung des Auges des Pobanden auf den in der Bildmitte dargestellten Fixationspunkt kann der Proband dem Arzt sehr schnell den Skotom-Bereich zeigen. Der Arzt kann daraufhin, beispielsweise mittels einer Maus den Skotom-Bereich in das Rauschbild einblenden, so wie dies in Fig. 3a dargestellt ist. Anschließend wird auf dem Bild ein Polarkoordinatenschema abgebildet¸ in welchem, wie in Fig. 3b dargestellt ist, das Skotom eingezeichnet erscheint. Anschließend ist es möglich, mit der statischen oder kinetischen Perimetrie auf homogenem Hintergrund diesen Bereich weiter zu untersuchen. Bei der kinetischen Perimetrie bewegt der Arzt den Prüfpunkt, beispielsweise mittels der Maus, frei über den Monitor (3c), wobei Gesichtsfeldausfälle durch Tastendruck des Probanden angezeigt werden können. Diese Vorgehensweise ist in Fig. 3c auf der unteren Bildhälfte dargestellt. Die statische Perimetrie erfolgt automatisch nach Art einer Rasterperimetrie, wobei die Prüfpunkte, wie in Fig. 3c in der oberen Fildhälfte dargestellt, in dem vorgewählten Gebiet durch Zufallsverteilung angeboten werden. Der Proband quittiert dabei gesehene Bildpunkte durch Knopfdruck.

Das erfindungsgemäße Verfahren schafft eine ausgezeichnete Screening-Methode, die es erlaubt, anschließend auf dem selben Monitor mit einer quantitativ messenden Perimetrie gezielt nur die pathologischen Bereiche im Gesichtsfeld zu untersuchen. Dadurch wird die Untersuchungszeit wesentlich kürzer als bei allen bisherigen Methoden.

Ein weiterer Vorteil ist die leichte und mühelose Handhabung der Methode. Arzt und Patient sitzen nebeneinander und betrachten gemeinsam den Monitorschirm, auf dem nacheinander oder in beliebiger Reihenfolge das Rauschfeld, das Polarkoordinatensystem und das Perimetriefeld erscheinen können. Die Gesichtsfelduntersuchung verliert mit dieser neuen Methode den Charakter der anstrengenden, ermüdenden Prüfung. Es kommt für den Patienten nicht mehr zu einer Streßsituation.

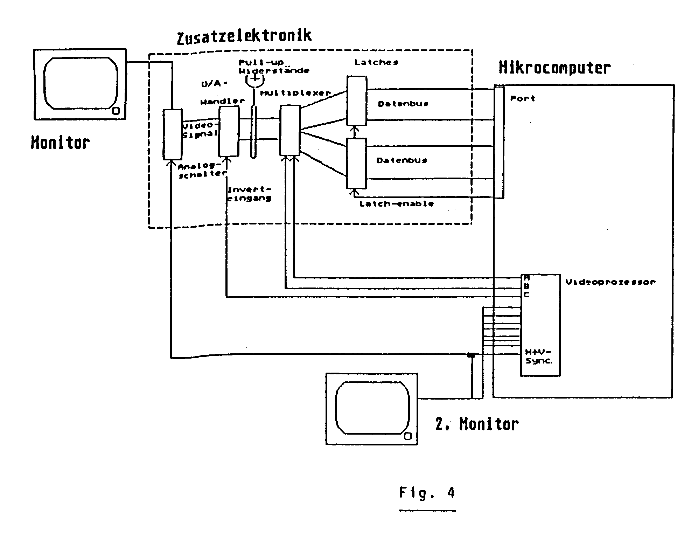

In Fig. 4 ist eine Vorrichtung für die Erzeugung eines schwellenperimetriegeeigneten Videobildes aus den Videosignalen eines Mikrocomputers dargestellt.

Es hat sich gezeigt, daß für ein schwellenperimetriegeeignetes Videobild nur zwei feinabgestufte Grauwerte und zwar für den Hintergrund und für den Prüfpunkt erforderlich sind, sowie ein wahlweise schwarzes oder weißes Fixationszeichen. Mit einer Zusatzelektronik für einen Mikrocomputer, kann ein schwellenperimetriegeeignetes Bild mit einfachen Mittel aus vier TTL-Videosignalen erzeugt werden. Voraussetzung ist hierbei, daß der Mikrocomputer ein aus mehr als 3 Bit bestehendes digitales Videosignal liefert. Der Vorteil dieses Verfahrens besteht in der Möglichkeit mit den verbleibenden Videosignalen des Computers ein weiteres schwarz-weißes Bild zu erzeugen z.B. auf einem zweiten Beobachtungsmonitor.

Die beiden abstufbaren Grauwerte werden als digitale Werte (Datenbus 8 oder mehr Bit, je nach der erforderlichen Zahl von Graustufen) vom Mikrocomputer in elektronischen Registern (Latches) gespeichert. Der Mikrocomputer stellt weiterhin vier TTL-Ausgänge seines Videoprozessors zur Verfügung. Mit einem ersten der TTL-Signale (A) kann ein Multiplexer geschaltet werden, der jeweils eines der elektronischen Register auf einen Digital-Analog-Wandler (D/A-Wandler) legt. Es kann also aus einem von einem Mikrocomputer erzeugten, und an den entsprechenden Videoausgängen anliegenden, binären Bild ein in seinen Grauwerten sehr stark variables Videobild erzeugt werden.

Ein zweites TTL-Signal (B) kann den Multiplexer tri-state schalten, so daß Pull-up-Widerstände, welche sich am Eingang des Digital-Analog-Wandlers befinden, den maximalen digitalen Wert an den Wandler legen. Mit Hilfe dieses zweiten TTL-Signales (B) kann eine Fixationszeichen eingeblendet werden, dessen Grauwert dem größten digitalen Eingangswert des Digital-Analog-Wandlers entspricht.

Soll das Fixationszeichen dem minimalen Digitalwert entsprechen, so ist dies mit Hilfe eines dritten TTL-Signales (C) möglich, wobei dieses dritte Signal ein im Digital-Analog-Wandler vorhandenes Invertierungsbit steuert. Hierdurch ist es möglich einen dem minimalen Digital-Wert entsprechendes Fixationszeichen darzustellen.

Der Digital-Analog-Wandler liefert ein analoges Signal, dem mit Hilfe eines Analogschalters die erforderlichen Videosynchronisationsimpulse beigemischt werden können. Diese Impulse werden ebenfalls vom Videoprozessor des Hostrechners als viertes TTL-Signal (D) übernommen.

## Patentansprüche

1. Vorrichtung zur Auffindung von Skotomen im Gesichtsfeld eines Probanden mit einem Schirm, und einer Einrichtung, mit der auf dem Schirm ein Bild erzeugbar ist, das eine Vielzahl dicht nebeneinanderliegender Bildpunkte aufweist, die Helligkeitsschwankungen unterworfen sind, dadurch gekennzeichnet, daß die Einrichtung geeignet ist, dicht nebeneinanderliegende schwarz/weiß oder farbige Bildpunkte zu erzeugen, deren Größe so wählbar ist, daß der Proband bei vorgegebener Sehleistung die einzelnen Bildpunkte aus einem vorgegebenen Abstand erkennen kann, deren Helligkeit statistisch oder periodisch mit einer derartig hohen Frequenz schwankt, daß die Bildpunkte dem Probanden flimmernd erscheinen, und daß die Einrichtung geeignet ist, zusätzlich einen Fixationspunkt (5) auf dem Schirm zu erzeugen.

2. Vorrichtung nach Anspruch 1, dadurch gekennzeichnet, daß der Fixationspunkt (5) horizontal wie auch vertikal auf dem Schirm zur Untersuchung zentraler oder peripherer Areale verschiebbar ist.

3. Vorrichtung nach einem der Ansprüche 1 oder 2, dadurch gekennzeichnet, daß benachbarte Bildpunkte einen hohen Kontrast zueinander aufweisen.

4. Vorrichtung nach einem der Ansprüche 1 bis 3, dadurch gekennzeichnet, daß mittels eines Lichtsensors ein integraler Helligkeitsabgleich des gesamten Bildes zur exakten Lichtunterschiedsempfindlichkeitsmessung pro Netzhautort durchführbar ist.

5. Vorrichtung nach einem der Ansprüche 1 bis 4, dadurch gekennzeichnet, daß das Bild nur auf einer vorbestimmten Fläche mit den Bildpunkten versehen ist.

6. Vorrichtung nach Anspruch 1 bis 5, dadurch gekennzeichnet, daß die Verteilung und/oder die Helligkeit der Bildpunkte mittels einer Zufallsfunktion oder eines Programms steuerbar ist.

7. Vorrichtung nach Anspruch 6, dadurch gekennzeichnet, daß die Frequenz des Helligkeitswechsels der Bildpunkte durch das Programm veränderbar ist.

8. Vorrichtung nach einem der Ansprüche 1 bis 7, dadurch gekennzeichnet, daß die Bildpunkte mit unterschiedlichen Farben oder mit wechselnden Farben ausgebildet sind.

9. Vorrichtung nach einem der Ansprüche 1 bis 8, dadurch gekennzeichnet, daß ein zusätzlicher Kontrollpunkt auf den blinden Fleck in Form und Lage des Probandenauges einstellbar ist.

10. Vorrichtung nach einem der Ansprüche 1 bis 9, dadurch gekennzeichnet, daß ein zweiter Schirm zur Kontrolle durch die behandelnde Person vorgesehen ist.

11. Verfahren zur Auffindung von Skotomen im Gesichtsfeld eines Probanden unter Verwendung einer Vorrichtung nach einem der Ansprüche 1 bis 10, dadurch gekennzeichnet, daß dem in einem vorgegebenen Abstand zu einem Schirm befindlichen Auge des Probanden auf dem Schirm ein Bild angeboten wird, das aus einer Vielzahl dicht nebeneinanderliegender Punkte besteht, deren Größe variabel ist und so gewählt wird, daß der Proband bei vorgegebener Sehleistung die einzelnen Bildpunkte aus einem vorgegebenen Abstand erkennen kann und deren Helligkeit statistisch oder periodisch mit einer derartig hohen Frequenz schwankt, daß die Bildpunkte dem Probanden flimmernd erscheinen, und das zusätzlich einen Fixationspunkt für das Auge des Probanden aufweist, wobei die Skotome vom Probanden als nicht flimmernde Flächen wahrgenommen und von ihm auf dem Schirm angezeigt werden.

12. Verfahren nach Anspruch 11, dadurch gekennzeichnet, daß die Helligkeit und die Helligkeitsschwankungen der einzelnen Bildpunkte einstellbar sind.

13. Verfahren nach einem der Ansprüche 11 oder 12, dadurch gekennzeichnet, daß dem Probanden ein reproduzierbares Bild angeboten wird, das mehr Dunkel- als Hellanteile aufweist.

14. Verfahren nach einem der Ansprüche 11 bis 13, dadurch gekennzeichnet, daß nach Durchführung der Verfahrensschritte nach den Ansprüchen 11 bis 13 auf dem Schirm zusätzlich dunkle Prüfpunkte auf hellem Untergrund oder helle Prüfpunkte auf dunklem Untergrund oder farbige Prüfpunkte erzeugt und gegebenenfalls verschoben werden zur Durchführung statischer oder kinetischer Perimetrie.

15. Verfahren nach Anspruch 14, dadurch gekennzeichnet, daß bei Verwendung eines planen Schirmes die Prüfpunkte in Größe und/oder Helligkeit je nach Lage auf dem Bildschirm anpaßbar sind, um gleiche Bedingungen wie in einer Perimeterhalbkugel zu schaffen.

16. Verfahren nach einem der Ansprüche 11 bis 13, dadurch gekennzeichnet, daß das Bild in eine Perimeterhalbkugel projiziert wird.

17. Verfahren nach einem der Ansprüche 11 bis 16, dadurch gekennzeichnet, daß die auf dem Schirm ermittelten Werte abgespeichert und gegebenenfalls ausgedruckt werden.

18. Verfahren nach einem der Ansprüche 11 bis 17, dadurch gekennzeichnet, daß auf dem Schirm ein Gesichtsfeldschema mit Gradangaben als Prüfkoordinatennetz eingeblendet wird.

19. Verfahren nach einem der Ansprüche 11 bis 18, dadurch gekennzeichnet, daß das Verfahren bei einem künstlich erhöhten Augendruck des Probanden durchgeführt wird.

20. Verfahren nach einem der Ansprüche 18 oder 19, dadurch gekennzeichnet, daß das Prüfpunktkoordinatennetz auf dem Monitor in Abhängigkeit vom variierbaren Abstand des untersuchten Auges zum Monitor jeweils umgerechnet und angepaßt dargestellt wird.

21. Verfahren nach einem der Ansprüche 11 bis 20, dadurch gekennzeichnet, daß das Patientenauge mit einer Kamera erfaßt und die Stellung des Auges auf einem Kontrollmonitor, vorteilhaft unter Einblendung eines Fadenkreuzes, dargestellt wird.

## Claims

1. A device for locating scotomas in the visual field of a patient using a screen, and apparatus by means of which an image can be generated on the screen, the image comprising a plurality of densely arranged image points which are subjected to brightness fluctuations, characterised in that the apparatus is suitable for generating densely arranged black/white or colour image points, the size of which can be selected such that the patient having a predetermined visual capacity can distinguish the individual image points from a predetermined distance, the brightness of the said image points fluctuating randomly or periodically with a level of frequency such that the image points appear to the patient to flicker, and in that the apparatus is suitable for also generating a fixation point (5) on the screen.

2. A device according to claim 1, characterised in that the fixation point (5) can be moved both horizontally and vertically on the screen in order to examine central or peripheral areas.

3. A device according to either one of claims 1 and 2, characterised in that adjacent image points have a high contrast in relation to each other.

4. A device according to any one of claims 1 to 3, characterised in that an integral brightness adjustment of the complete image can be carried out by means of a light sensor in order to measure precisely the light contrast sensitivity per retinal site.

5. A device according to any one of claims 1 to 4, characterised in that the image is only provided by the image points over a predetermined area.

6. A device according to claims 1 to 5, characterised in that the distribution and/or the brightness of the image points can be controlled by means of a random function or a program.

7. A device according to claim 6, characterised in that the frequency of the change in brightness of the image points can be altered by the program.

8. A device according to any one of claims 1 to 7, characterised in that the image points are of different colours or of changing colour.

9. A device according to any one of claims 1 to 8, characterised in that an additional monitoring point can be adjusted to the blind spot of the patient's eye in shape and position.

10. A device according to any one of claims 1 to 9, characterised in that a second screen which is to be monitored by the person giving the treatment is provided.

11. A method of locating scotomas in the visual field of a patient using a device according to any one of claims 1 to 10, characterised in that an image on a screen is presented to the patient's eye which is positioned at a predetermined distance from the screen, the image comprising a plurality of densely arranged points, the size of which is variable and selected such that the patient having a predetermined visual capacity can distinguish the individual image points from a predetermined distance, and the brightness of the said points fluctuating randomly or periodically with a level of frequency such that the image points appear to the patient to flicker, and the image additionally having a fixation point for the patient's eye, the scotomas of the patient being perceived as non-flickering areas and being indicated by him on the screen.

12. A method according to claim 11, characterised in that the brightness and the brightness fluctuations of the individual image points are adjustable.

13. A method according to either one of claims 11 and 12, characterised in that a reproducible image which has more dark areas than light areas is presented to the patient.

14. A method according to any one of claims 11 to 13, characterised in that after carrying out the procedural steps according to claims 11 to 13, dark test points on a light background or light test points on a dark background or coloured test points are additionally generated on the screen and if necessary shifted in order to carry out static or kinetic perimetry.

15. A method according to claim 14, characterised in that when using a flat screen, the test points are adjustable in size and/or brightness according to their position on the screen in order to create the same conditions as in a perimeter hemisphere.

16. A method according to any one of claims 11 to 13, characterised in that the image is projected into a perimeter hemisphere.

17. A method according to any one of claims 11 to 16, characterised in that the values determined on the screen are stored and if necessary printed out.

18. A method according to any one of claims 11 to 17, characterised in that a visual field diagram with indications of degree is superimposed on the screen as a test co-ordinate grid.

19. A method according to any one of claims 11 to 18, characterised in that the patient's eye pressure is artificially increased when carrying out the method.

20. A method according to either one of claims 18 and 19, characterised in that the test point co-ordinate grid on the monitor is changed as a function of the variable distance of the examined eye from the monitor and displayed accordingly.

21. A method according to any one of claims 11 to 20, characterised in that the patient's eye is detected by a camera and the position of the eye is shown on a control monitor, advantageously by superimposing cross-hairs.

## Revendications

1. Dispositif pour trouver des scotomes dans le champ de vision d'un patient, comportant un écran et un appareillage permettant de reproduire sur cet écran une image qui présente un grand nombre de points d'image disposés côte à côte et très serrés, et soumis aux variations de l'intensité lumineuse; caractérisé en ce que l'appareillage peut faire apparaître des points d'image très serrés, en noir/blanc ou en couleur et de grandeur réglable, de telle sorte qu'avec une acuité visuelle donnée et à une certaine distance, le patient peut identifier ceux de ces points d'image d'écartement donné à l'avance dont la luminosité est soumise à une variation statistique ou périodique d'une fréquence telle qu'il lui apparaissent sous forme de scintillements; en outre, cet appareillage est apte à reproduire un point de fixation (5) sur l'écran.

2. Dispositif selon la revendication 1, caractérisé en ce que le point de fixation (5) est déplaçable sur l'écran, dans le sens horizontal comme dans le sens vertical, pour l'exploration des zones centrale ou périphérique.

3. Dispositif selon l'une des revendications 1 ou 2, caractérisé en ce que les points d'image contigus sont fortement contrastés entre eux.

4. Dispositif selon l'une des revendications 1 à 3, caractérisé en ce qu'il permet d'obtenir une égalisation intégrale de l'intensité lumineuse sur l'ensemble de l'image pour mesurer ainsi de façon exacte la sensibilité aux contrastes lumineux par zone rétinienne explorée.

5. Dispositif selon l'une des revendications 1 à 4, caractérisé en ce que, seule une surface déterminée de la figure représentée est couverte des points d'image.

6. Dispositif selon les revendications 1 à 5, caractérisé en ce que la répartition et/ou la luminosité des points d'image est réglable au moyen d'une fonction aléatoire ou d'un programme.

7. Dispositif selon la revendication 6, caractérisé en ce que la fréquence de variation de la luminosité des points d'image est modifiable grâce au programme.

8. Dispositif selon l'une des revendications 1 à 7, caractérisé en ce que les points d'image sont représentés dans des nuances différentes ou changeantes.

9. Dispositif selon l'une des revendications 1 à 8, caractérisé en ce qu'un point de contrôle supplémentaire est réglable sur la tache aveugle, et suivant la forme et la position de l'oeil du patient.

10. Dispositif selon l'une des revendications 1 à 9, caractérisé en ce qu'un second écran de visualisation est prévu pour le contrôle par la personne à traiter elle-même.

11. Procédé pour trouver des scotomes dans le champ visuel d'un patient par utilisation d'un dispositif selon l'une des revendications 1 à 10, caractérisée en ce qu'une image est proposée sur un écran à l'oeil du patient se trouvant à une distance donnée à l'avance de cet écran; cette image se composant d'un très grand nombre de points disposés côte à côte et très serrés, dont la grandeur est variable et est choisie de telle sorte que le patient ayant une acuité visuelle fixée à l'avance puisse, à une distance donnée, identifier séparément ces points d'image, dont la luminosité est soumise à des variations statistiques ou périodiques d'une fréquence telle qu'ils lui apparaissent comme des scintillements, l'image comportant en outre un point de fixation pour l'oeil du patient, lequel peut ainsi observer les scotomes sous forme de taches exemptes de scintillement et les indiquer sur l'écran.

12. Procédé selon la revendication 11, caractérisé en ce que la luminosité et les variations d'intensité lumineuse de chacun des points d'image sont réglables.

13. Procédé selon l'une des revendications 11 ou 12, caractérisé en ce qu'une image reproductible, présentant plus de parties sombres que de parties claires, est proposée au patient.

14. Procédé selon l'une des revendications 11 à 13, caractérisé en ce que des points de contrôle sont en outre produits sur l'écran, après les étapes des processus selon les revendications 11 à 13, ces points de contrôle étant de couleur sombre sur fond clair ou clairs sur fond sombre ou colorés, et, sont éventuellement décalés pour constituer une périmétrie statique ou cinétique.

15. Procédé selon la revendication 14, caractérisé en ce que les points de contrôle sont adaptables en grandeur et/ou en luminosité, et suivant les nécessités, à l'écran si celui-ci est plat, ceci, afin de créer des conditions identiques à celles que pose un hémisphère périmétrique.

16. Procédé selon l'une des revendications 11 à 13, caractérisé en ce que l'image est projetée dans un hémisphère périmétrique.

17. Procédé selon l'une des revendications 11 à 16, caractérisé en ce que les valeurs déterminées sur l'écran sont mémorisées et éventuellement imprimées.

18. Procédé selon l'une des revendications 11 à 17, caractérisé en ce qu'un schéma du champ visuel gradué apparaît en surimpression sur l'écran, en tant que système de coordonnées pour le contrôle.

19. Procédé selon l'une des revendications 11 à 18, caracatérisé en ce qu'il est mis en oeuvre alors que la pression oculaire du patient est augmentée artificiellement.

20. Procédé selon l'une des revendications 18 ou 19, caractérisé en ce que le système de coordonnées du point de contrôle est représenté sur le moniteur, après avoir été transformé et adapté en fonction de la distance variable entre l'oeil exploré et le moniteur.

21. Procédé selon l'une des revendications 11 à 20, caractérisé en ce que l'oeil du patient est exploré au moyen d'une caméra, et que sa position est représentée sur un moniteur de contrôle, de façon avantageuse grâce à l'insertion d'un réticule dans le dispositif.
